(19) Europäisches Patentamt
European Patent Office
Office européen des brevets

(11) **EP 3 906 838 A1**

(12) **EUROPEAN PATENT APPLICATION**
published in accordance with Art. 153(4) EPC

(43) Date of publication:
**10.11.2021 Bulletin 2021/45**

(51) Int Cl.:
**A61B 1/313** (2006.01)　　　**G02B 13/00** (2006.01)

(21) Application number: **19907894.0**

(22) Date of filing: **26.12.2019**

(86) International application number:
**PCT/CN2019/128912**

(87) International publication number:
**WO 2020/140833 (09.07.2020 Gazette 2020/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.01.2019　CN 201910004069**

(71) Applicant: **Eaglescope Medical Technology Co. Ltd**
**Changzhou, Jiangsu 213000 (CN)**

(72) Inventors:
• ZHANG, Jianzhong
  hangzhou, Jiangsu 213000 (CN)
• SUN, Shaopeng
  hangzhou, Jiangsu 213000 (CN)
• YANG, Changrong
  hangzhou, Jiangsu 213000 (CN)

(74) Representative: **Patentanwälte Gierlich & Pischitzis**
**Partnerschaft mbB**
**Gerbermühlstraße 11**
**60594 Frankfurt am Main (DE)**

(54) **4K LAPAROSCOPE OBJECTIVE LENS WITH 30° VIEWING ANGLE**

(57) The present invention relates to an objective lens for 4K laparoscope with a field direction angle of 30° including a concave meniscus lens, a prism assembly, a triple cemented lens, a cemented lens, a first rear meniscus lens and a second rear meniscus lens, which are cemented sequentially along the light propagation direction; wherein the concave meniscus lens has a convex surface on one side and an even aspheric surface on the other side, the triple cemented lens has a flat surface on one side and a convex surface on the other side, the cemented lens has a convex surface on each of the both sides, and both of the first rear meniscus lens and the second rear meniscus lens have a concave surface on one side and a convex surface on the other side; the even aspheric surface of the concave meniscus lens is cemented to one side of the prism assembly, the other side of the prism assembly is cemented to the flat surface of the triple cemented lens, the convex surface of the triple cemented lens is cemented to the convex surface on one side of the cemented lens, the convex surface on the other side of the cemented lens is cemented to the first rear meniscus lens, and the concave surface of the first rear meniscus lens and the concave surface of the second rear meniscus lens are cemented together oppositely. The present invention applies to laparoscope with a field direction angle of 30°, and can realize an ultra-high-definition resolution of 4K.

Fig. 5

**Description**

FIELD OF THE INVENTION

The present invention relates to an objective lens, in particular to an objective lens for 4K ultra-high-definition laparoscope with a field direction angle of 30°.

BACKGROUND ART

In minimally invasive procedures, all image information is collected through an endoscope. Endoscopes with higher resolution and sharper images can not only improve the safety of surgery, but also enrich the options for surgery.

The rigid laparoscope is composed of three parts: objective, relay lens and eyepiece. The function of the objective lens is to image the observed object surface onto the intermediate image plane in front of the relay lens. The relay lens is composed of 3 or 5 groups of rod lenses with a magnification of -1, which can not only transfer the image formed by the objective lens to the outside, but also realize the function of balancing the aberration. The eyepiece magnifies the image transmitted by the relay lens, which is convenient for visual observation or connection with the adapter of the endoscope.

At present, many endoscope designs are provided in the prior art, which can be roughly divided into two categories: 1) optical designs of rigid tube endoscopes, and 2) optical designs of electronic endoscopes. The biggest difference between these two is that: the rear end of the rigid tube endoscope needs to be connected with 3 to 5 groups of relay lenses, thus the objective lens utilizes a image-side telecentric structure; while in the electronic endoscope, the image plane of the objective lens is linked to CCD or CMOS, thus the image-side telecentricity is not required, and the design difficulty is relatively low. In the international standards, the rigid tube endoscope has a field direction angle of 0°, 12°, 30°, 45° or 70°. In the design of a laparoscope with a field direction angle of 0°, there is no need to consider the problem of light interference in the turning of the prism. However, in the design of a laparoscope with a field direction angle of 30°, the problem of light interference is a technical difficulty that must be overcome. In this sense, the design of the laparoscope with a field direction angle of 30° has some new features over that of the laparoscope with a field direction angle of 0°.

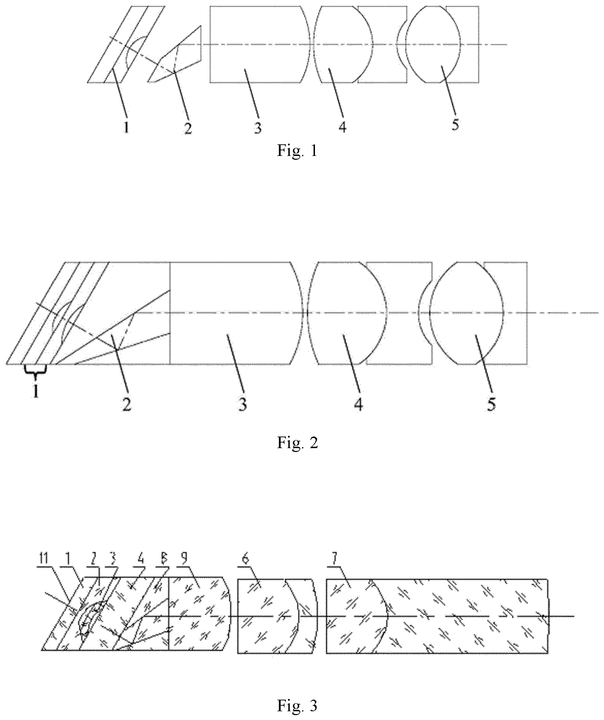

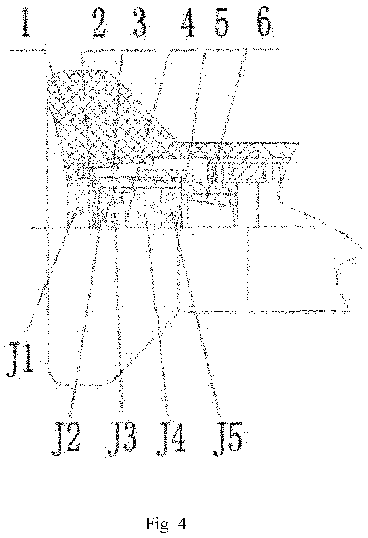

There are various designs for objective lens in the prior art. First, in designs as shown in Figs. 1 and 2, 1 or 2 plano-concave lenses, a turning prism assembly and a plano-convex lens are used, and these three types of lenses are glued together. In the practical design of the structure in Fig. 1, it was found that a single prism limits the relative aperture of the objective lens, making it difficult to achieve high-definition resolution. In the structure of Fig. 2, two plano-concave lenses and a prism assembly are used; however, the variables in the design are still insufficient, and it is difficult to achieve 4K resolution. Second, as shown in Fig. 3, except for the negative lens necessary for the reversed telephoto structure at the front end, this design almost consists of cemented lenses with positive focal power, which obviously violates the principle of phase difference balance in optical design, and it is difficult to achieve higher resolution. At the same time, the last lens is obviously too long, and the front end is concave and has a relatively steep surface; therefore, the processing of the lens is relatively difficult, which can neither solve the problem, but also increase the cost. Third, as shown in Fig. 4, the eyepiece is set as an aspheric surface to balance the residual phase difference of the entire lens. However, this design does not take into account that the objective lens is located in the back half of the endoscope during adjustment, and needs to be adjusted to fill the view field of the eyepiece. Therefore, it is difficult for the eyepiece, objective lens and rod lens to be coaxial, thus the theoretical phase difference balance cannot be achieved.

SUMMARY OF THE INVENTION

The objective of the present invention is to provide an objective lens for 4K laparoscope with a field direction angle of 30°, which is applicable for laparoscope with a field direction angle of 30° and can achieve an ultra-high-definition resolution of 4K.

In order to achieve the above objective, the present invention provides an objective lens for 4K laparoscope with a field direction angle of 30°, and its inventive point lies in that: it comprises a concave meniscus lens, a prism assembly, a triple cemented lens, a cemented lens, a first rear meniscus lens and a second rear meniscus lens, which are cemented sequentially along the light propagation direction.

The concave meniscus lens has a convex surface on one side and an even aspheric surface on the other side, the triple cemented lens has a flat surface on one side and a convex surface on the other side, the cemented lens has a convex surface on each of the both sides, and both of the first rear meniscus lens and the second rear meniscus lens have a concave surface on one side and a convex surface on the other side.

The even aspheric surface of the concave meniscus lens is cemented to one side of the prism assembly, the other side of the prism assembly is cemented to the flat surface of the triple cemented lens, the convex surface of the triple cemented lens is cemented to the convex surface on one side of the cemented lens, the convex surface on the other side of the cemented lens is cemented to the first rear meniscus lens, and the concave surface of the first rear meniscus lens and the concave surface of the second rear meniscus lens are cemented together oppositely.

In the above technical solution, the convex surface on one side of the concave meniscus lens has a curvature of 0.025 to 1, and the even aspheric surface on the other side of the concave meniscus lens has a curvature of 0.33 to 1.

In the above technical solution, the triple cemented lens is formed by cementing three convex lenses with high-temperature-resistant UV photosensitive glue, and the UV photosensitive glue can withstand a high temperature of 140°C to 160°C.

In the above technical solution, the cemented lens is a doublet lens which is formed by cementing two lenses with high-temperature-resistant UV photosensitive glue, and the UV photosensitive glue can withstand a high temperature of 140°C to 160°C.

In the above technical solution, the cemented lens is a triplet lens which is formed by cementing three lenses with high-temperature-resistant UV photosensitive glue, and the UV photosensitive glue can withstand a high temperature of 140°C to 160°C.

In the above technical solution, the concave meniscus lens, the prism assembly, the triplet lens, the cemented lens, the first rear meniscus lens and the second rear meniscus lens are sequentially cemented with UV photosensitive glue, methanol glue or optical epoxy glue.

In the above technical solution, the curvature radius of the concave surface on one side of the first rear meniscus lens is smaller than the curvature radius of the convex surface on the other side of the first rear meniscus lens, and the first rear meniscus lens has a thickness of 4 mm to 8 mm. The curvature radius of the concave surface on one side of the second rear meniscus lens is smaller than the curvature radius of the convex surface on the other side of the second rear meniscus lens, and the second rear meniscus lens has a thickness of 4 mm to 6 mm.

The objective lens of the present invention comprises a concave meniscus lens, a prism assembly, a triple cemented lens, a cemented lens, a first rear meniscus lens and a second rear meniscus lens, which are cemented sequentially along the light propagation direction; wherein the concave meniscus lens has a convex surface on one side and an even aspheric surface on the other side, the triple cemented lens has a flat surface on one side and a convex surface on the other side, the cemented lens has a convex surface on each of the both sides, and both of the first rear meniscus lens and the second rear meniscus lens have a concave surface on one side and a convex surface on the other side; and wherein the even aspheric surface of the concave meniscus lens is cemented to one side of the prism assembly, the other side of the prism assembly is cemented to the flat surface of the triple cemented lens, the convex surface of the triple cemented lens is cemented to the convex surface on one side of the cemented lens, the convex surface on the other side of the cemented lens is cemented to the first rear meniscus lens, and the concave surface of the first rear meniscus lens and the concave surface of the second rear meniscus lens are cemented together oppositely.

Therefore, the present invention has the following benefits: after using the objective lens for 4K laparoscope with a field direction angle of 30°, the laparoscope when in use has an object distance in a range of 10 mm to 500 mm, and a view field in a range of 70° to 80°; and the size of the primary image plane, which is limited by the diameter of the inner tube of the endoscope, is generally less than 5 mm, thus the focal length is about 3 mm to 3.5 mm. Since a large incident angle will introduce large high-level aberrations, the first lens of the inventive objective lens adopts a concave meniscus lens. One side of the concave meniscus lens is a convex surface, which can reduce the incident angle of the main rays of the large view field, thereby avoiding the influence of stray light caused by the glancing incidence and reducing high-level aberrations. The other side of the concave meniscus lens is an even aspheric surface. Since the aberration characteristics of incident beams are different in different view fields, the introduction of aspheric surfaces can differentially balance the aberrations of different view fields, which helps to achieve 4K resolution.

The turning angle of the prism assembly is two folds of the angle between the two mirror planes reflected inside the prism. Therefore, the desired field direction angle of 30° is achieved by controlling the angle between the two planes inside the prism to be 15°.

The lens on the other side of the prism assembly is a triplet cemented lens. The material of the cemented lens connected with the prism assembly is consistent with that of the prism assembly. The flat surface on one side of the triple cemented lens is convenient for cementing to the prism assembly. Provided behind the triple cemented lens is a cemented lens, both sides of which are convex surfaces. These two cemented lenses will be used to balance the chromatic aberration of the system.

At the end of the objective lens, two rear meniscus lenses are used, and the concave surfaces thereof are arranged oppositely. Although they are negative lenses, they have positive refractive power. The setting of the two rear meniscus lenses helps to achieve a larger positive field curvature of the system.

The present invention applies to laparoscope with a field direction angle of 30°, and can realize an ultra-high-definition resolution of 4K.

BRIEF DESCRIPTION OF THE DRAWINGS

Fig. 1 is a schematic diagram of the first kind of structure of an objective lens for laparoscope in the prior art;
Fig. 2 is a schematic diagram of the second kind of structure of an objective lens for laparoscope in the prior art;
Fig. 3 is a schematic diagram of the structure of a laparoscope in the prior art;
Fig. 4 is a schematic diagram of the structure of an eyepiece for laparoscope in the prior art; and
Fig. 5 is a schematic diagram of the structure of an embodiment of the present invention.

DETAILED DESCRIPTION OF THE INVENTION

The present invention will be further described below in conjunction with, but not limited to, the accompanying drawings and the preferred embodiments.

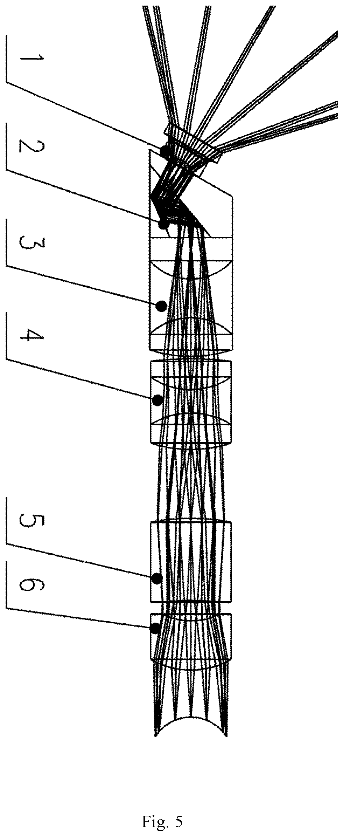

As shown in Fig. 5, an objective lens for 4K laparoscope with a field direction angle of 30° includes a concave meniscus lens 1, a prism assembly 2, a triple cemented lens 3, a cemented lens 4, a first rear meniscus lens 5 and a second rear meniscus lens 6, which are cemented sequentially along the light propagation direction;

the concave meniscus lens 1 has a convex surface on one side and an even aspheric surface on the other side, the triple cemented lens 3 has a flat surface on one side and a convex surface on the other side, the cemented lens 4 has a convex surface on each of the both sides, and both of the first rear meniscus lens 5 and the second rear meniscus lens 6 have a concave surface on one side and a convex surface on the other side; and

the even aspheric surface of the concave meniscus lens 1 is cemented to one side of the prism assembly 2, the other side of the prism assembly 2 is cemented to the flat surface of the triple cemented lens 3, the convex surface of the triple cemented lens 3 is cemented to the convex surface on one side of the cemented lens 4, the convex surface on the other side of the cemented lens 4 is cemented to the first rear meniscus lens 5, and the concave surface of the first rear meniscus lens 5 and the concave surface of the second rear meniscus lens 6 are cemented together oppositely.

In the present invention, the convex surface on one side of the concave meniscus lens 1 has a curvature of 0.025 to 1, and the even aspheric surface on the other side of the concave meniscus lens 1 has a curvature of 0.33 to 1. The conic coefficient $k$ ranges from -0.1 to -1.5, $\alpha_1$=0, $\alpha_2$ ranges from -1.0e-3 to -1.0e-4, and $\alpha_3$ ranges from -1.0e-4 to -1.0e-5. The aspheric surface satisfies the following equation:

$$z = \frac{cr^2}{1 + \sqrt{1 - (1 + k)c^2 r^2}} + \alpha_1 r^2 + \alpha_2 r^4 + \alpha_3 r^6$$

wherein:

$z$ is the vector height of the aspheric surface;
$r$ is the height of the annulus;
$c$ is the curvature;
$k$ is the conic coefficient; and
$\alpha_1$, $\alpha_2$ and $\alpha_3$ are the second-, fourth- and sixth-order coefficients, respectively.

In the present invention, the triple cemented lens 3 is formed by cementing three convex lenses with high-temperature-resistant UV photosensitive glue, and the UV photosensitive glue can withstand a high temperature of 140°C to 160°C.

In the present invention, the cemented lens 4 is a doublet lens which is formed by cementing two lenses with high-temperature-resistant UV photosensitive glue, and the UV photosensitive glue can withstand a high temperature of 140°C to 160°C.

In the present invention, the cemented lens 4 is a triple cemented lens which is formed by cementing three lenses with high-temperature-resistant UV photosensitive glue, and the UV photosensitive glue can withstand a high temperature of 140°C to 160°C.

The refractive index of the triple cemented lens glass connected to the prism needs to satisfy the following requirements:

$$n_1 - n_2 \geq 0.2$$

$$n_3 - n_2 \geq 0.08$$

$$v_1 \leq 20$$

$$v_2 \leq 35$$

$$v_3 \leq 50$$

wherein $n_1$, $n_2$ and $n_3$ are the refractive indexes of the three glasses at the wave length of 589.3 nm, respectively; and $v_1$, $v_2$ and $v_3$ are the Abbe numbers of the three glasses, respectively.

In the present invention, the concave meniscus lens 1, the prism assembly 2, the triple cemented lens 3, the cemented lens 4, the first rear meniscus lens 5 and the second rear meniscus lens 6 are sequentially cemented with UV photosensitive glue, methanol glue or optical epoxy glue.

In the present invention, the curvature radius of the concave surface on one side of the first rear meniscus lens 5 is smaller than the curvature radius of the convex surface on the other side of the first rear meniscus lens 5, and the first rear meniscus lens 5 has a thickness of 4 mm to 8 mm. The curvature radius of the concave surface on one side of the second rear meniscus lens 6 is smaller than the curvature radius of the convex surface on the other side of the second rear meniscus lens 6, and the second rear meniscus lens 6 has a thickness of 4 mm to 6 mm.

In the present invention, the image plane of the objective lens adopts the design of positive field curvature. Preferably, the image plane has a curvature radius in a range of 2 mm to 10 mm. The curvature direction of the image plane is in the direction of the objective lens. At the same time, the objective lens still satisfies the requirements of image-side telecentricity.

In information theory, the imaging optical system is a low-pass filter. The design of 4K resolution requires the imaging lens to have better performance in the high-frequency information part. The design of the traditional laparoscope objective lens with a diameter of less than 6 mm cannot meet the design requirements of 4K resolution; compared to that, the objective lens of the present invention preferably has a diameter of 6.5 mm to 10 mm.

Example 1

The object surface beam passes through the concave meniscus lens, which significantly reduces the incident angle. Then, the object surface beam passes through the prism assembly to complete the setting of the field direction angle. Next to that, it passes through the triple cemented lens and the doublet lens to balance chromatic aberration. Finally, it passes through two rear meniscus lenses to achieve positive field curvature.

The parameters are shown in Table 1.

Table 1

| Surface No. | Curvature Radius | Thickness | $N_d/V_d$ | Diameter | Notes |
|---|---|---|---|---|---|
| 1 | 65.6 | 1 | 1.81, 40.9 | 4.2 | |
| 2 | 1.5 | 0.58 | air | 3 | K=-0.7 |
| 3 | -- | 8.4 | 1.517, 64.1 | | prism assembly being equivalent to parallel plates |
| 4 | - | 3.3 | 1.96, 17.5 | 7 | triple cemented |
| 5 | -4.43 | 4.9 | 1.62, 36.4 | 7 | |
| 6 | 4.991 | 3.4 | 1.83,29.2 | 7 | |

(continued)

| Surface No. | Curvature Radius | Thickness | $N_d/V_d$ | Diameter | Notes |
|---|---|---|---|---|---|
| 7 | -9.627 | 2.9 | air | | |
| 8 | 33.58 | 1.9 | 1.85, 24.8 | 7 | double cemented |
| 9 | 5.71 | 3 | 1.58, 59.4 | 7 | |
| 10 | -18.713 | 0.43 | air | | |
| 11 | 11.602 | 7.8 | 1.62, 53.3 | 7 | |
| 12 | 12.798 | 1.7 | air | 6.5 | |
| 13 | -5.57 | 5.628 | 1.49, 70.2 | 6.4 | |
| 14 | -6.58 | 3.718 | | 7 | |
| IMA | 4.5 | | | | positive field curvature |

In the present invention, the introduction of two sets of cemented lenses plays a key role in the balance of chromatic aberration. In traditional designs, a rear meniscus lens is usually used at the end of the system to achieve a flat field, while the present invention uses two rear meniscus lenses to achieve positive field curvature.

Example 2

The object surface beam passes through the meniscus lens, which significantly reduces the incident angle. Then, the object surface beam passes through the prism assembly to complete the setting of the field direction angle. Next to that, it passes through the triple cemented lens and another triple cemented lens to balance chromatic aberration. Finally, it passes through two rear meniscus lenses to achieve positive field curvature.
The parameters are shown in Table 2.

Table 2

| Surface No. | Curvature Radius | Thickness | $N_d/V_d$ | Notes |
|---|---|---|---|---|
| 1 | 28.9 | 1 | 1.81, 40.9 | |
| 2 | 1.4 | 0.61 | air | K=-0.6 |
| 3 | -- | 9.1 | 1.517, 64.1 | prism assembly being equivalent to parallel plates |
| 4 | - | 3.5 | 1.96, 17.5 | triple cemented |
| 5 | -4.68 | 3.3 | 1.64, 42.4 | |
| 6 | 5 | 3.3 | 1.70,41.2 | |
| 7 | 26.5 | 0.2 | air | |
| 8 | -5.88 | 2.6 | 1.61, 56.7 | triple cemented |
| 9 | 6.5 | 2 | 1.85, 24.8 | |
| 10 | -10.74 | 3.1 | 1.61, 56.7 | |
| 11 | 17.19 | 5.86 | air | |
| 12 | 21.46 | 7 | 1.96, 17.5 | |
| 13 | -5.6 | 1.73 | air | |
| 14 | -6.78 | 4.2 | 1.59,61.1 | |
| IMA | 3.5 | 4 | | positive field curvature |

In minimally invasive procedures using a laparoscope, the insufflator needs to be used in conjunction with the laparoscope to form a cavity, which not only ensures that the laparoscope can have a larger view field, but also ensures that the instrument does not collide and interfere with the laparoscope when in service. The shape of the cavity produced by $CO_2$ is close to a sphere, thus the object surface in this design is a spherical surface. The radius of the sphere is consistent with the object distance. Theoretically, the imaging optical system adopts plane-to-plane imaging. However, the object surface as a curved surface will increase the difficulty of aberration balance; therefore, a triple cemented solution is used for 8 to 11 surfaces in the present design to increase variables and thus to provide additional support for aberration balance.

When in use, the laparoscope has an object distance in the range of 10 mm to 500 mm, and a view field in the range of 70° to 80°. The size of the primary image plane, which is limited by the diameter of the inner tube of the endoscope, is generally less than 5 mm, thus the focal length is about 3 mm to 3.5 mm. Since a large incident angle will introduce large high-level aberrations, the first lens of the inventive objective lens adopts a concave meniscus lens. One side of the concave meniscus lens is a convex surface, which can reduce the incident angle of the main rays of the large view field, thereby avoiding the influence of stray light caused by the glancing incidence and reducing high-level aberrations. The other side of the concave meniscus lens is an even aspheric surface. Since the aberration characteristics of incident beams are different in different view fields, the introduction of aspheric surfaces can differentially balance the aberrations of different view fields, which helps to achieve 4K resolution.

The turning angle of the prism assembly is two folds of the angle between the two mirror planes reflected inside the prism. Therefore, the desired field direction angle is achieved by controlling the angle between the two planes inside the prism.

The lens on the other side of the prism assembly is a triplet cemented lens. The material of the cemented lens connected with the prism assembly is consistent with that of the prism assembly. The flat surface on one side of the triple cemented lens is convenient for cementing to the prism assembly. Provided behind the triple cemented lens is a cemented lens, both sides of which are convex surfaces. These two cemented lenses will be used to balance the chromatic aberration of the system.

At the end of the objective lens, two rear meniscus lenses are used, and the concave surfaces thereof are arranged oppositely. Although they are negative lenses, they have positive refractive power. The setting of the two rear meniscus lenses helps to achieve a large positive field curvature of the system.

The present invention applies to laparoscope with a field direction angle of 30°, and can realize an ultra-high-definition resolution of 4K.

Based on the teachings of the above-mentioned preferred embodiments of the present invention, those skilled in the art can make various changes and modifications without departing from the spirit of the present invention. The protection scope of the present invention is determined by the claims, and is not limited by the specification.

**Claims**

1.  An objective lens for 4K laparoscope with a field direction angle of 30°, comprising a concave meniscus lens (1), a prism assembly (2), a triple cemented lens (3), a cemented lens (4), a first rear meniscus lens (5) and a second rear meniscus lens (6), which are cemented sequentially along the light propagation direction;
    wherein the concave meniscus lens (1) has a convex surface on one side and an even aspheric surface on the other side, the triple cemented lens (3) has a flat surface on one side and a convex surface on the other side, the cemented lens (4) has a convex surface on each of both sides, and both of the first rear meniscus lens (5) and the second rear meniscus lens (6) have a concave surface on one side and a convex surface on the other side; and
    wherein the even aspheric surface of the concave meniscus lens (1) is cemented to one side of the prism assembly (2), the other side of the prism assembly (2) is cemented to the flat surface of the triple cemented lens (3), the convex surface of the triple cemented lens (3) is cemented to the convex surface on one side of the cemented lens (4), the convex surface on the other side of the cemented lens (4) is cemented to the first rear meniscus lens (5), and the concave surface of the first rear meniscus lens (5) and the concave surface of the second rear meniscus lens (6) are cemented together oppositely.

2.  The objective lens for 4K laparoscope with a field direction angle of 30° according to claim 1, **characterized in that**: the convex surface on one side of the concave meniscus lens (1) has a curvature of 0.025 to 1, and the even aspheric surface on the other side of the concave meniscus lens (1) has a curvature of 0.33 to 1.

3.  The objective lens for 4K laparoscope with a field direction angle of 30°according to claim 1, **characterized in that**: the triple cemented lens (3) is formed by cementing three convex lenses with high-temperature-resistant UV photosensitive glue, and the UV photosensitive glue can withstand a high temperature of 140°C to 160°C.

4.  The objective lens for 4K laparoscope with a field direction angle of 30°according to claim 1, **characterized in that**:

the cemented lens (4) is a doublet lens which is formed by cementing two lenses with high-temperature-resistant UV photosensitive glue, and the UV photosensitive glue can withstand a high temperature of 140°C to 160°C.

5. The objective lens for 4K laparoscope with a field direction angle of 30°according to claim 1, **characterized in that**: the cemented lens (4) is a triple cemented lens which is formed by cementing three lenses with high-temperature-resistant UV photosensitive glue, and the UV photosensitive glue can withstand a high temperature of 140°C to 160°C.

6. The objective lens for 4K laparoscope with a field direction angle of 30°according to claim 1 or 4, **characterized in that**: the concave meniscus lens (1), the prism assembly (2), the triple cemented lens (3), the cemented lens (4), the first rear meniscus lens (5) and the second rear meniscus lens (6) are sequentially cemented with UV photosensitive glue, methanol glue or optical epoxy glue.

7. The objective lens for 4K laparoscope with a field direction angle of 30°according to claim 1, **characterized in that**:

the curvature radius of the concave surface on one side of the first rear meniscus lens (5) is smaller than the curvature radius of the convex surface on the other side of the first rear meniscus lens (5), and the first rear meniscus lens (5) has a thickness of 4 mm to 8 mm; and
the curvature radius of the concave surface on one side of the second rear meniscus lens (6) is smaller than the curvature radius of the convex surface on the other side of the second rear meniscus lens (6), and the second rear meniscus lens (6) has a thickness of 4 mm to 6 mm.

Fig. 1

Fig. 2

Fig. 3

Fig. 4

Fig. 5

## INTERNATIONAL SEARCH REPORT

| International application No. |
| --- |
| **PCT/CN2019/128912** |

| | |
| --- | --- |
| **A.   CLASSIFICATION OF SUBJECT MATTER** | |
| A61B 1/313(2006.01)i;   G02B 13/00(2006.01)i | |
| According to International Patent Classification (IPC) or to both national classification and IPC | |

**B.   FIELDS SEARCHED**

Minimum documentation searched (classification system followed by classification symbols)

A61B; G02B

Documentation searched other than minimum documentation to the extent that such documents are included in the fields searched

Electronic data base consulted during the international search (name of data base and, where practicable, search terms used)

CNABS, CNTXT, VEN: 腹腔镜, 硬镜, 硬管, 内窥镜, 物镜, 透镜组, 高清, 分辨率, 干涉, 视向角, 棱镜, 色差, 非球面, 光焦度, 场曲 laparoscope, endoscope, surgical, hard, catheter, tube, object lens, lens set, high ressolution, retina, interference, asphere, view, angle, prism, color difference, deviation, deflection, focal power, field, curvature

**C.   DOCUMENTS CONSIDERED TO BE RELEVANT**

| Category* | Citation of document, with indication, where appropriate, of the relevant passages | Relevant to claim No. |
| --- | --- | --- |
| PX | CN 109620103 A (EAGLESCOPE MEDICAL TECHNOLOGY CO. LTD.) 16 April 2019 (2019-04-16)<br>claims 1-7 | 1-7 |
| PX | CN 209595703 U (EAGLESCOPE MEDICAL TECHNOLOGY CO. LTD.) 08 November 2019 (2019-11-08)<br>claims 1-7 | 1-7 |
| Y | 张树青 等 (ZHANG, Shuqing et al.). "30°视向角硬质内窥镜光学设计 (Optical Design of Rigid Endoscope with 30° Viewing Angle)"<br>光学学报 (Acta Optica Sinica), Vol. 38, No. no. 02, 28 February 2018 (2018-02-28),<br>pp. 0222002-1 to 0222002-6 | 1-7 |
| Y | CN 107102433 A (JIANGSU (CHINA) EAGLESCOPE MEDICAL EQUIPMENT LIMITED COMPANY) 29 August 2017 (2017-08-29)<br>description, paragraphs 19-31, figures 1-3 | 1-7 |
| A | CN 106580236 A (TIANJIN UNIVERSITY) 26 April 2017 (2017-04-26)<br>entire document | 1-7 |

☑ Further documents are listed in the continuation of Box C.       ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **16 March 2020** | **23 March 2020** |

| Name and mailing address of the ISA/CN | Authorized officer |
| --- | --- |
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2019/128912**

### C.    DOCUMENTS CONSIDERED TO BE RELEVANT

| Category* | Citation of document, with indication, where appropriate, of the relevant passages | Relevant to claim No. |
|---|---|---|
| A | CN 104905759 A (QINGDAO AOMEIKE MEDICAL TECHNOLOGY CO LTD) 16 September 2015 (2015-09-16) <br> entire document | 1-7 |
| A | CN 107085295 A (JIANGSU (CHINA) EAGLESCOPE MEDICAL EQUIPMENT LIMITED COMPANY) 22 August 2017 (2017-08-22) <br> entire document | 1-7 |
| A | CN 206450895 U (JIANGSU (CHINA) EAGLESCOPE MEDICAL EQUIPMENT LIMITED COMPANY) 29 August 2017 (2017-08-29) <br> entire document | 1-7 |
| A | CN 106597645 A (JIANGSU (CHINA) EAGLESCOPE MEDICAL EQUIPMENT LIMITED COMPANY) 26 April 2017 (2017-04-26) <br> entire document | 1-7 |
| A | US 2012127598 A1 (OLYMPUS MEDICAL SYSTEMS CORP et al.) 24 May 2012 (2012-05-24) <br> entire document | 1-7 |
| A | US 2007258150 A1 (TAKATO HIDEYASU) 08 November 2007 (2007-11-08) <br> entire document | 1-7 |

Form PCT/ISA/210 (second sheet) (January 2015)

## INTERNATIONAL SEARCH REPORT
### Information on patent family members

| International application No. |
|---|
| **PCT/CN2019/128912** |

| Patent document cited in search report | | | Publication date (day/month/year) | Patent family member(s) | | | Publication date (day/month/year) |
|---|---|---|---|---|---|---|---|
| CN | 109620103 | A | 16 April 2019 | None | | | |
| CN | 209595703 | U | 08 November 2019 | None | | | |
| CN | 107102433 | A | 29 August 2017 | WO | 2019001274 | A1 | 03 January 2019 |
| | | | | CN | 107102433 | B | 20 August 2019 |
| CN | 106580236 | A | 26 April 2017 | CN | 106580236 | B | 17 April 2018 |
| CN | 104905759 | A | 16 September 2015 | WO | 2015135390 | A1 | 17 September 2015 |
| | | | | US | 9895050 | B2 | 20 February 2018 |
| | | | | CN | 104905759 | B | 18 January 2017 |
| | | | | DE | 112015000729 | T5 | 29 December 2016 |
| | | | | US | 2016174808 | A1 | 23 June 2016 |
| CN | 107085295 | A | 22 August 2017 | WO | 2019001275 | A1 | 03 January 2019 |
| CN | 206450895 | U | 29 August 2017 | None | | | |
| CN | 106597645 | A | 26 April 2017 | None | | | |
| US | 2012127598 | A1 | 24 May 2012 | WO | 2011125539 | A1 | 13 October 2011 |
| | | | | JP | WO2011125539 | A1 | 08 July 2013 |
| | | | | US | 8300325 | B2 | 30 October 2012 |
| | | | | JP | 4902033 | B1 | 21 March 2012 |
| US | 2007258150 | A1 | 08 November 2007 | JP | 5371178 | B2 | 18 December 2013 |
| | | | | US | 7511892 | B2 | 31 March 2009 |
| | | | | JP | 2007260305 | A | 11 October 2007 |

Form PCT/ISA/210 (patent family annex) (January 2015)